# FASCICULE DE BREVET EUROPEEN

(11) **EP 2 334 647 B1**
(45) Date de publication et mention de la délivrance du brevet: **22.03.2017**
(21) Numéro de dépôt: 09748401.8
(22) Date de dépôt: 17.09.2009
(51) Int. Cl.: C07D 233/16, C09K 8/54, C23F 11/14

(54) **INHIBITEURS DE CORROSION PEU TOXIQUES ET BIODEGRADABLES**
BIOLOGISCH ABBAUBARE KORROSIONSINHIBITOREN MIT NIEDRIGER TOXIZITÄT
LOW-TOXICITY BIODEGRADABLE CORROSION INHIBITORS

(30) Priorité: 18.09.2008 FR 0856293
(43) Date de publication de la demande: 22.06.2011
(73) Titulaire: CECA S.A., 92250 La Garenne Colombes (FR)
(72) Inventeur: POU, Tong Eak, F-69540 Irigny (FR); GILLET, Jean-Philippe, F-69530 Brignais (FR); GANCET, Christian, F-64140 Lons (FR)
(74) Mandataire: Lhoste, Catherine
(86) Numéro de dépôt international: PCT/FR2009/051745
(87) Numéro de publication internationale: WO 2010/031963

(56) Documents cités:
- EP-A- 0 526 251
- EP-A- 1 043 423
- WO-A-98/41673
- DE-A1- 2 003 175
- GB-A- 2 319 530
- US-A- 2 865 817
- QURAISHI M A ET AL: "Synthesis and evaluation of some organic vapour phase corrosion inhibitors" INDIAN JOURNAL OF CHEMICAL TECHNOLOGY, SCIENTIFIC PUBLISHERS, JODHPUR, INDIA, IN, vol. 11, no. 4, 1 janvier 2004 (2004-01-01), pages 459-464, XP009115025 ISSN: 0971-457X
- QURAISHI M A ET AL: "Inhibition of metallic corrosion by some 1-(2-aminoethyl)-2-undecyl-2 imidazoline salts under vapor phase conditions" BULLETIN OF ELECTROCHEMISTRY, KARAIKUDI, vol. 19, no. 7, 1 janvier 2003 (2003-01-01), pages 295-300, XP009115023 ISSN: 0256-1654
- ISAGULYANTS V I ET AL: "Synthesis of diimidazoline salts of dicarboxylic acids which are corrosion inhibitors" KORROZIYA I ZASHCHITA V NEFTEGAZOVOI PROMYSHLENNOSTI,, vol. 7, 1 janvier 1971 (1971-01-01), pages 3-4, XP009115022

## Description

La présente invention concerne des composés inhibiteurs de corrosion des métaux qui sont peu toxiques et biodégradables. L'invention concerne également l'utilisation de ces composés inhibiteurs de corrosion notamment dans l'industrie pétrolière, et plus généralement tout type d'industrie de forage de minerais ou de composés fossiles, tels que le gaz ou le pétrole.

Dans la production pétrolière ou gazière, la corrosion des matériaux constituant les installations de forage, tels que plate-formes, canalisations, vannes et autres équipements, est un véritable problème qui nécessite de nombreuses opérations de maintenance et de réparation. La corrosion des métaux dans ces catégories d'industrie représente par conséquent un coût très important.

L'utilisation d'inhibiteurs de corrosion est souvent une solution économique intéressante. Cependant les inhibiteurs de corrosion, outre leur propriété intrinsèque anti-corrosion, ne doivent pas avoir un effet néfaste pour l'environnement.

Les molécules chimiques connues aujourd'hui pour être de bons inhibiteurs de corrosion pour lutter contre la corrosion carbonique (due au CO₂) ou la corrosion sulfhydrique (due à l'H₂S) sont le plus souvent des imidazolines, des amines et dérivées, des sels d'ammonium quaternaires et des esters phosphoriques. Ces molécules souffrent cependant d'un inconvénient majeur en ce qu'elles sont néfastes pour l'environnement.

Il est également connu d'apporter des modifications de structure chimique de certaines molécules organo-azotées pour les rendre moins toxiques, comme décrit par exemple par J. P. Clewlow et coll. (brevet US 5,427,999) qui ont décrit la réduction de la toxicité des amines ou des imidazolines par réaction avec des acides acryliques. Selon R. L. Martin et coll. (brevet US 5,785,895), l'association entre une N-éthoxy-imidazoline substituée en position 2 et un phosphate-ester obtenu à partir de l'acide phosphorique et d'un alcool en C₈-C₁₀ (Alfol 8-10) éthoxylé, conduit à un composé qui est peu toxique.

A. Naraghi et coll (brevet US 6,475,431) enseignent que les molécules, issues de la réaction entre une amido-amine, un acide carboxylique insaturé (tel que l'acide acrylique) et l'acide monochloracétique sont efficaces contre la corrosion pétrolière et peu toxiques.

Les travaux de QURAISHI MA et coli. ("Synthesis and evaluation of some organic vapour phase corrosion inhibitors", INDIAN JOURNAL OF CHEMICAL TECHNOLOGY, SCIENTIFIC PUBLISHERS, JODHPUR, INDIA, IN, (2004), vol. 11, no. 4, ISSN 0971-457X, PAGE 459 - 464, XP009115025) décrivent le maléate de 2-déc-9-ényl-2-imidazoline, qui est utilisé comme inhibiteur de corrosion.

D'autres travaux de QURAISHI MA et coll. ("Inhibition of metallic corrosion by some 1-(2-aminoethyl)-2-undecyl-2-imidazoline salts under vapor phase conditions", BULLETIN OF ELECTROCHEMISTRY, KARAIKUDI, (2003), vol. 19, no. 7, ISSN 0256-1654, pp. 295-300, XP009115023) décrivent le maléate de N-aminoéthyl-2-undécénylimidazoline comme inhibiteur de corrosion.

ISAGULYANTS VI et coll. ("Synthesis of diimidazoline salts of dicarboxylic acids which are corrosion inhibitors", KORROZIYA I ZASHCHITA V NEFTEGAZOVOI PROMYSHLENNOSTI, (1971), vol. 7, p. 3-4, XP009115022) décrivent quant à eux un sébaçate d'imidazolium utile comme inhibiteur de corrosion.

Le document EP0526251 décrit des composés contenant un groupe (CH₂)₁₋₄CO₂H pouvant comporter un reste imidazoline.

Le document WO1998041673 décrit une composition inhibitrice de corrosion du fer comprenant un dérivé d'imidazoline et un mercaptoacide.

Le document DE2003175 décrit un procédé pour préparer un sel d'imidazoline résultant de l'addition d'un acide pouvant être choisi parmi l'acide maléique ou fumarique.

Le document EP1043423 décrit une composition inhibitrice de corrosion comprenant un groupe imidazoline, mais pas de sel de diacide d'imidazoline.

Le document US2865817 décrit des composés utilisés pour rendre moins corrosif le procédé de refroidissement lors de la préparation du coke , lesdits composés comprenant un dérivé d'imidazoline tel que (N-aminoéthyl-2-heptadécénylimidazoline et acide sébacique), (N-aminoéthyl-2-heptadécénylimidazoline et acide citrique), (N-aminoéthyl-2-tridécénylimidazoline et acide sébacique), (2-heptadécénylimidazoline et acide subérique).

Le document GB2319530 décrit une composition inhibitrice de corrosion comprenant un dérivé d'amine qui peut être un groupe imidazoline, mais pas de sel de diacide d'imidazoline.

Il subsiste toutefois un besoin pour des composés anti-corrosion encore plus efficaces, encore moins toxiques et surtout présentant une biodégradabilité élevée.

La demanderesse a ainsi découvert de manière tout à fait inattendue qu'il est possible d'augmenter la biodégradabilité et de diminuer la toxicité d'un composé de type imidazoline, tout en maintenant de bonnes propriétés anti-corrosion, notamment pour les diverses installations utilisées dans les industries pétrolières et gazières.

Ainsi, selon un premier aspect, la présente invention concerne une formulation comprenant au moins un carboxylate d'imidazoline de formule (1) : 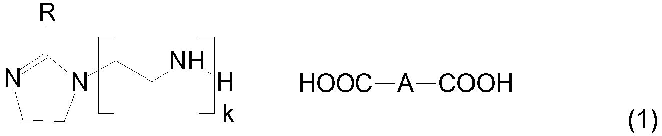 dans laquelle :
- R représente un radical hydrocarboné, saturé ou insaturé, linéaire ou ramifié, de préférence linéaire, comportant 17 atomes de carbone,
- k représente 1, 2, 3 ou 4,
- A représente -CH=CH-,
et au moins un solvant choisi de préférence parmi l'eau, les alcools, les glycols, et les mélanges de deux ou plusieurs d'entre eux en toutes proportions.

Le terme « insaturé » utilisé dans la définition des carboxylates (ou sels) d'imidazoline de formule (1) ci-dessus, indique la présence d'une ou plusieurs insaturations sous forme de double(s) et/ou triple(s) liaison(s), de préférence sous forme de double(s) liaison(s).

On préfère les carboxylates de formule (1) ci-dessus présentant une, de préférence plusieurs, de préférence encore toutes, les caractéristiques suivantes prises isolément ou en combinaison :
- R représente un radical hydrocarboné, saturé ou insaturé, linéaire ou ramifié, de préférence linéaire, comportant 17 atomes de carbone,
- k représente 1 ou 2,
- A représente -CH=CH-.

Selon un aspect encore plus préféré, les sels (carboxylates) d'imidazoline définis ci-dessus sont des carboxylates de N-aminoéthyl-2-heptadécénylimidazoline. De manière tout à fait préférée, les carboxylates selon l'invention est le maléate, de N-aminoéthyl-2-heptadécénylimidazoline.

Les sels d'imidazoline (1) selon la présente invention sont avantageusement obtenus par salification d'au moins un dérivé d'imidazoline de formule (1a) : 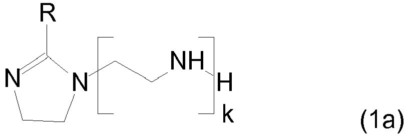 avec au moins un diacide carboxylique de formule (1 b) :

HOOC-A-COOH (1 b)

formules (1 a) et (1 b) dans lesquelles R, k et A sont tels que définis précédemment.

La réaction de salification peut être conduite selon toute méthode couramment utilisée et connue de l'homme du métier. Les carboxylates d'imidazoline de formule (1) peuvent par exemple être aisément obtenus par mise en contact d'au moins un dérivé d'imidazoline de formule (1a) avec au moins un diacide carboxylique de formule (1 b), puis chauffage du mélange réactionnel, sous agitation.

La température de réaction peut varier dans de grandes proportions, selon la nature des dérivés d'imidazoline et des diacides employés. Le solvant de réaction peut être l'eau, un ou plusieurs solvants, de préférence hydrosolubles, ou encore un mélange eau/solvant(s) hydrosoluble(s).

Les dérivés d'imidazoline de formule (1a) pouvant être utilisés pour la synthèse des sels de formule (1) sont soit connues, disponibles dans le commerce, ou facilement préparées à partir de modes opératoires connus ou adaptés de modes opératoires connus et disponibles dans la littérature scientifique, la littérature brevet, les « Chemical Abstracts » ou encore sur Internet.

Selon un mode de réalisation préféré, les dérivés d'imidazoline (1a) sont choisis parmi les akylimidazolines, et de préférence est la N-(aminoéthyl)aminoéthyl-2-heptadécénylimidazoline et la N-aminoéthyl-2-heptadécénylimidazoline, ainsi que les mélanges de deux ou plusieurs alkylimidazolines. Selon un mode de réalisation tout à fait préféré, le dérivé d'imidazoline de formule (1A) est la N-aminoéthyl-2-heptadécénylimidazoline (ou 2-{2-[(8*E*)-heptadéc-8-ényl]-4,5-d ihydro-1*H*-imidazol-1-yl}éthanamine).

À titre d'exemple non limitatif, la N-aminoéthyl-2-heptadécénylimidazoline peut avantageusement être obtenue par réaction cyclisante entre la DETA (diéthylène triamine) et l'acide oléique. Les sels de formule (1) selon la présente invention présente ainsi l'avantage de pouvoir être préparés, en totalité ou en partie, à partir de matières renouvelables, et notamment à partir d'acides gras présents dans la nature, tels que l'acide oléique précédemment cité.

Parmi les diacides carboxyliques de formule (1b) qui peuvent être utilisés pour la préparation des sels de formule (1), on peut citer l'acide maléïque

(HOOC-CH=CH-COOH).

Comme indiqué ci-dessus, les sels de formule (1) selon la présente invention peuvent être obtenus par réaction entre au moins un dérivé d'imidazoline de formule (1a) et au moins un diacide de formule (1 b).

Le ratio molaire dérivé d'imidazoline (1a)/diacide (1b) est généralement compris entre 1/0,1 et 1/5, de préférence entre 1/0,5 et 1/3, plus préférentiellement entre 1/1 et 1/2.

Selon un autre objet, la présente invention concerne l'utilisation d'une formulation telle qu'elle vient d'être définie en tant que inhibiteurs de corrosion dans tout type d'industrie de forage de minerais ou de composés fossiles, et notamment dans les industries pétrolière et gazière, et plus généralement comme inhibiteurs de corrosion des conduites transportant le pétrole brut ou le gaz.

En tant qu'inhibiteurs de corrosion, les formulations selon l'invention peuvent être utilisées dans un solvant ou un mélange de solvants hydrosolubles, et de préférence peu toxique(s) et biodégradable(s). Les solvants pouvant être utilisés sont, à titre d'exemples non limitatifs, les solvants hydrosolubles, tels que l'eau, les alcools, les glycols, et plus précisément l'eau, le méthanol, l'éthanol, le mono-éthylèneglycol, et les mélanges de deux ou plusieurs d'entre eux en toutes proportions.

Ainsi, les formulations selon l'invention peuvent être dans l'eau, ou encore dans un ou plusieurs solvants organiques ou encore dans l'eau avec un ou plusieurs solvants organiques (formulation hydro-organique).

De manière tout à fait avantageuse, la formulation forme une formulation anti-corrosion compatible avec l'environnement. Selon un mode de réalisation préféré, la présente invention concerne une formulation comprenant de 1% à 90%, de préférence de 10% à 30% en poids d'au moins un sel de formule (1), de 0% à 20%, de préférence de 1% à 10% en poids d'au moins un tensioactif, avantageusement compatible avec l'environnement, et le complément à 100% en poids d'au moins un solvant (eau, solvant(s) organique(s) ou hydro-organique(s)).

La formulation décrite ci-dessus peut elle-même être utilisée telle quelle ou encore être diluée, par exemple juste avant emploi, dans de l'eau et/ou dans un ou plusieurs solvants, de préférence un ou plusieurs alcools, tels que méthanol, éthanol et/ou mono-éthylène glycol.

Les tensioactifs utilisables dans la formulation selon la présente invention peuvent être de tout type parmi ceux connus de l'homme du métier, non-ioniques, ioniques ou amphotères.

Les formulations selon la présente invention sont très efficaces en tant qu'inhibiteurs de corrosion dans tout type d'industrie de forage de minerais ou de composés fossiles, tels que le gaz ou le pétrole, et en particulier dans les industries pétrolière et gazière.

Les fluides transportés dans les canalisations, vannes, pompes et autres, sont des milieux très corrosifs, en raison de la présence d'une quantité plus ou moins importante d'eau saturée en dioxyde de carbone (CO₂) et/ou sulfure d'hydrogène (H₂S).

Les formulations de l'invention peuvent être utilisées pour le traitement par injection en continu, par batch ou par « squeeze », dans les fluides transportés dans les divers conduits, vannes, pompes, etc. d'une installation de forage.

Selon encore un autre objet, la présente invention concerne le procédé pour éviter ou limiter la corrosion carbonique (due au CO₂ dissous dans l'eau) et/ou la corrosion sulfhydrique (due au H₂S dissous dans l'eau) des parties métalliques, en particulier en acier, susceptibles d'être dégradées par corrosion carbonique et/ou par corrosion sulfhydrique, ledit procédé comprenant la mise en contact des dites parties métalliques avec au moins une formulation telle que définie précédemment comprenant carboxylate d'imidazoline.

La quantité d'inhibiteur(s) de corrosion utilisée peut varier dans de grandes proportions, notamment en fonction du type de traitement à réaliser. D'une manière générale et non limitative, cette quantité est avantageusement comprise entre 1 ppm et 10% (poids/volume) par rapport au volume de fluide transporté.

Plus précisément, la quantité de composé inhibiteur de corrosion peut par exemple être comprise entre 2 ppm et 50 ppm (poids/volume) pour l'injection continue (injection en surface), entre 100 ppm et 1% (poids/volume) pour le traitement par batch (par bouchon pour filmer la paroi de canalisation), et de 1% à 10% (poids/volume) pour le traitement par squeeze (injection au fond du puits de pétrole jusqu'à la formation).

Les exemples qui suivent sont fournis à titre d'illustration et n'ont pas pour but de limiter la portée de la présente invention définie par les revendications annexées.

### Exemple 1 : Préparation des composés de l'invention (Procédé général)

La synthèse des composés est effectuée par réaction entre un diacide carboxylique avec un dérivé d'imidazoline. À titre d'exemple, le dérivé d'imidazoline peut être la N-aminoéthyl-2-heptadécénylimidazoline, elle-même obtenue à partir d'acide oléïque et de diéthylènetriamine (DETA) selon des procédés classiques connus de l'homme du métier.

La réaction peut se faire par ajout direct du diacide solide sur l'imidazoline substituée, mais également, notamment pour des raisons liées à la viscosité du milieu, on peut utiliser une solution ou une suspension du diacide dans l'éthylèneglycol qui est coulé sur l'imidazoline.

Les ratios molaires diacide/dérivé d'imidazoline sont compris entre 1/1 et 2/1.

### Exemple 2 : Préparation du maléate de N-aminoéthyl-2-heptadécénylimidazoline

On prépare une suspension de 34,7 g (0,3 mole) d'acide maléïque dans 69 g d'éthylèneglycol. Cette suspension est coulée sur 103 g (0,3 mole) de N-aminoéthyl-2-heptadécénylimidazoline (imidazoline A), disponible chez CECA, et maintenue à 40°C dans un réacteur avec agitation mécanique. On poursuit ensuite l'agitation à cette température pendant 2 heures. On obtient ainsi une huile visqueuse homogène à 64,5% d'extrait sec.

De manière similaire, les composés des exemples 3 à 6, qui ne font pas partie de l'invention, sont obtenus, en faisant varier la nature du diacide dicarboxylique. Ces composés sont obtenus à environ 50% en poids dans le mono-éthylène glycol. Les composés des exemples 3 à 6 sont listés dans le Tableau 1 suivant :

**-- Tableau 1 --**

| ***Exemple n°*** | ***Sel d'imidazoline* / *diacide carboxylique*** |
|---|---|
| 3 | Imidazoline A / Acide succinique (1/1) |
| 4 | Imidazoline A / Acide malique (1/1) |
| 5 | Imidazoline A / Acide tartrique (1/1) |
| 6 | Imidazoline A / Acide glutarique (1/1) |

### Exemple 7 : Écotoxicité des molécules étudiées

### a) Toxicité algale

La toxicité des substances pour l'environnement peut être mesurée sur différents tests normalisés. Un des plus sensibles est constitué par la mesure de la toxicité sur algues d'eau douce (*Pseudokirchneriella subcapitata*). Le test est réalisé selon la Ligne Directrice 201 de l'OCDE. Il consiste à évaluer l'inhibition de la croissance des algues sur une durée de 72 heures. Le paramètre caractéristique est la CE₅₀ qui est la concentration de la substance provoquant une inhibition de 50 % de la croissance algale au cours du test.

Le tableau 2 ci-après donne les CE₅₀ des produits testés :

**-- Tableau 2 --**

| **Composés** | ***Toxicité algale (CE₅₀ en mg*/*L)*** |
|---|---|
| Imidazoline A à 50% en poids dans le mono-éthylèneglycol | 0,0047 |
| Exemple 2 | 1 |
| Exemple 3 | 0,13 |
| Exemple 4 | 0,29 |
| Exemple 5 | 0,14 |

### 2) Biodégradabilité marine

Les tests de biodégradabilité sont effectués en milieu marin selon la Ligne Directrice OCDE 306. Les résultats obtenus sont donnés dans le tableau 3 ci-après :

**-- Tableau 3 --**

| ***Composés*** | ***Biodégradabilité OCDE 306, % de biodégradation en 28 jours*** |
|---|---|
| Imidazoline A à 50% en poids dans le mono-éthylèneglycol | 57 |
| Exemple 2 | 66 |
| Exemple 3 | 62 |
| Exemple 5 | 59 |

De manière inattendue, les composés de l'invention qui sont des sels de diacides carboxyliques de dérivés d'imidazoline sont nettement moins toxiques que l'imidazoline seule non-salifiée (facteur 30 à 200) et que leur biodégradabilité tout à fait comparable, voire améliorée.

### Exemple 8 : Vitesses de corrosion en absence et en présence des composés de l'invention

### 8.1. Corrosion carbonique

Les vitesses de corrosion carbonique sont mesurées par la méthode de mesure de résistance de polarisation utilisant une cellule de corrosion à double enveloppe comprenant un système à trois électrodes (électrode test en acier au carbone, électrode référence en calomel saturée et contre-électrode en platine) dans les conditions suivantes :

### a) Milieu corrosif biphasique :

20% de White Spirit
80% de solution à 1 g/L de chlorure de sodium (NaCl)

Le mélange ci-dessus (milieu corrosif biphasique) est désaéré, par barbotage d'azote, puis saturé en dioxyde de carbone (CO₂) par barbotage de ce gaz. Le mélange est ensuite introduit dans la cellule de corrosion à double enveloppe décrite précédemment.

La température de travail est de 80°C. Le dosage est de 25 ppm (volume/volume) de composé à tester à 50% en poids d'extrait sec dans le mono éthylène glycol par rapport au milieu biphasique : on ajoute dans un volume de 1 litre de milieu corrosif (20% de White Spirit + 80% de solution aqueuse à 1 g/L de chlorure de sodium), 25 microlitres de la formule tester.

Une fois que tout est en place, l'électrode test, l'électrode de référence et la contre-électrode dans la phase aqueuse et à 80°C sous agitation magnétique à environ 100 tours par minute, la formule testée est injectée dans la phase huile (White Spirit). On suit l'évolution de la vitesse de corrosion de l'électrode test, dans la phase aqueuse, pendant au moins 2 heures c'est-à-dire jusqu'à stabilisation avec le temps.

Une fois la vitesse de corrosion du témoin stabilisée (vitesse de corrosion de l'acier au carbone sans inhibiteur c'est à dire avant l'ajout d'inhibiteur de corrosion), on introduit 25 ppm en volume de composé à tester dans la phase huile et on suit la vitesse de corrosion de l'électrode test dans la phase aqueuse.

Les résultats sont donnés dans le tableau 4.

**-- Tableau 4 --**

| **Composé à tester** | **Vcor (mm/an)** |
|---|---|
| Témoin | 3,5 |
| Référence (imidazoline A à 50% en poids dans le mono éthylène glycol) | 0,04 |
| Exemple 2 | 0,05 |
| Exemple 3 | 0,07 |
| Exemple 4 | 0,08 |
| Exemple 5 | 0,1 |
| Exemple 6 | 0,08 |

Les composés de l'invention (sels obtenus par réaction entre les diacides carboxyliques et les dérivés d'imidazoline) montrent une efficacité contre la corrosion carbonique tout à fait comparable à celle observée avec l'utilisation des dérivés d'imidazoline seuls.

### 8.2. Corrosion sulfhydrique,

On répète les opérations décrites dans l'exemple 8.1 de corrosion carbonique, le milieu corrosif biphasique (White Spirit et solution à 1 g/L de NaCl) étant cette fois-ci saturée en sulfure d'hydrogène (H₂S).

Les résultats obtenus sont rassemblés dans le tableau 5.

**-- Tableau 5 --**

| **Composé à tester** | **Vcor (mm/an)** |
|---|---|
| Témoin | 2,5 |
| Référence (imidazoline A à 50% en poids dans le mono éthylène glycol) | 0,04 |
| Exemple 2 | 0,02 |
| Exemple 3 | 0,05 |
| Exemple 4 | 0,07 |
| Exemple 5 | 0,08 |
| Exemple 6 | 0,06 |

Les composés de l'invention (sels obtenus par réaction entre les diacides carboxyliques et les dérivés d'imidazoline) montrent une efficacité contre la corrosion sulfhydrique tout à fait comparable à celle observée avec l'utilisation des dérivés d'imidazoline seuls.

## Revendications

1. Formulation comprenant au moins un carboxylate d'imidazoline de formule (1) : 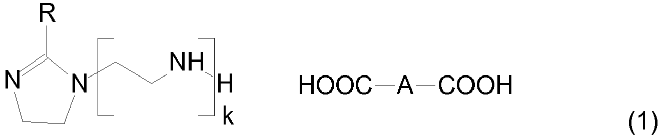 dans laquelle :
• R représente un radical hydrocarboné, saturé ou insaturé, linéaire ou ramifié, de préférence linéaire, comportant 17 atomes de carbone,
• k représente 1, 2, 3, ou 4,
• A représente -CH=CH-,
et au moins un solvant choisi de préférence parmi l'eau, les alcools, les glycols, et les mélanges de deux ou plusieurs d'entre eux en toutes proportions.

2. Formulation selon la revendication 1 comprenant :
- de 1 % à 90%, de préférence de 10% à 30% en poids d'au moins un carboxylate selon la revendication 1,
- de 0% à 20%, de préférence de 1% à 10% en poids d'au moins un tensioactif,
- et le complément à 100% en poids d'au moins un solvant.

3. Utilisation d'au moins une formulation selon l'une quelconque des revendications 1 à 2, comme inhibiteur de corrosion dans tout type d'industrie de forage de minerais ou de composés fossiles, et notamment dans les industries pétrolière et gazière, et plus généralement comme inhibiteur de corrosion de conduites transportant le pétrole brut ou le gaz.

4. Procédé pour éviter ou limiter la corrosion carbonique et/ou la corrosion sulfhydrique des parties métalliques, en particulier en acier, susceptibles d'être dégradées par corrosion carbonique et/ou par corrosion sulfhydrique, ledit procédé comprenant la mise en contact desdites parties métalliques avec au moins une formulation selon l'une quelconque des revendications 1 ou 2.

5. Procédé selon la revendication 4, dans lequel la formulation comprenant le carboxylate est injectée en continu, par batch ou par « squeeze », dans les fluides transportés dans les divers conduits, vannes, pompes, etc. d'une installation de forage, en particulier de pétrole ou de gaz.

6. Procédé selon la revendication 4 ou la revendication 5, dans lequel la quantité de carboxylate d'imidazoline est avantageusement comprise entre 1 ppm et 10% (poids/volume) par rapport au fluide transporté, de préférence entre 2 ppm et 50 ppm (poids/volume) pour l'injection continue, entre 100 ppm et 1% (poids/volume) pour le traitement par batch, et de 1% à 10% (poids/volume) pour le traitement par squeeze.

## Patentansprüche

1. Formulierung, die mindestens ein Imidazolincarboxylat der Formel (1) umfasst: 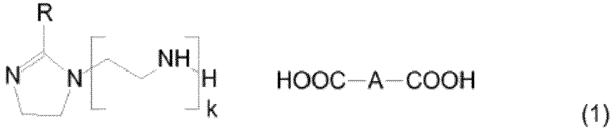 wobei:
• R für einen Kohlenwasserstoffrest steht, der gesättigt oder ungesättigt, geradkettig oder verzweigt, vorzugsweise geradkettig ist, wobei er 17 Kohlenstoffatome aufweist,
• k für 1, 2, 3 oder 4 steht,
• A für -CH=CH- steht,
sowie mindestens ein Lösungsmittel, das vorzugsweise aus Wasser, Alkoholen, Glykolen und Mischungen von zweien oder mehreren davon in beliebigen Anteilen ausgewählt ist.

2. Formulierung nach Anspruch 1, umfassend:
- 1 % bis 90 %, vorzugsweise 10 % bis 30 % nach Gewicht mindestens eines Carboxylats nach Anspruch 1,
- 0 % bis 20%, vorzugsweise 1 % bis 10 % nach Gewicht mindestens eines Tensids,
- sowie den zu 100 % nach Gewicht fehlenden Anteil an mindestens einem Lösungsmittel.

3. Verwendung mindestens einer Formulierung nach einem beliebigen der Ansprüche 1 bis 2 als Korrosionsinhibitor in einem beliebigen Wirtschaftszweig der Förderung von Erzen oder fossilen Verbindungen, und insbesondere in der Erdöl- und Erdgaswirtschaft, sowie allgemeiner ausgedrückt als Korrosionsinhibitor für Leitungen, die Rohöl oder Gas transportieren.

4. Verfahren zum Verhindern oder Begrenzen der kohlendioxidbedingten Korrosion und/oder der schwefelwasserstoffbedingten Korrosion von Metallteilen, insbesondere aus Stahl, die durch kohlendioxidbedingte Korrosion und/oder schwefelwasserstoffbedingte Korrosion beschädigt werden könnten, wobei das Verfahren insbesondere das Inkontaktbringen der Metallteile mit mindestens einer Formulierung nach einem beliebigen der Ansprüche 1 oder 2 umfasst.

5. Verfahren nach Anspruch 4, wobei die Formulierung, welche das Carboxylat umfasst, kontinuierlich, im Batch- oder im "Squeeze"-Verfahren in die Fluids eingeleitet wird, welche in den verschiedenartigen Leitungen, Ventilen, Pumpen usw. einer Anlage zur Förderung, insbesondere von Erdöl oder von Gas, transportiert werden.

6. Verfahren nach Anspruch 4 oder Anspruch 5, wobei die Menge an Imidazolincarboxylat vorteilhafterweise im Bereich von 1 ppm bis 10 % (Gewicht/Volumen) unter Bezugnahme auf das transportierte Fluid liegt, und zwar vorzugsweise von 2 ppm bis 50 ppm (Gewicht/Volumen) für die kontinuierliche Einleitung, von 100 ppm bis 1 % (Gewicht/Volumen) für die Behandlung im Batch-Verfahren, und von 1 % bis 10 % (Gewicht/Volumen) für die Behandlung im Squeeze-Verfahren.

## Claims

1. Formulation comprising at least one imidazoline carboxylate of formula (1): 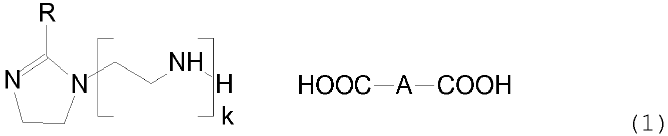 in which:
• R represents a saturated or unsaturated and linear or branched, preferably linear, hydrocarbon radical comprising 17 carbon atoms,
• k represents 1, 2, 3 or 4,
• A represents -CH=CH-,
and at least one solvent preferably chosen from water, alcohols, glycols and the mixtures of two or more of them in all proportions.

2. Formulation according to Claim 1, comprising:
- from 1% to 90% by weight, preferably from 10% to 30% by weight, of at least one carboxylate according to Claim 1,
- from 0% to 20% by weight, preferably from 1% to 10% by weight, of at least one surfactant,
- and the remainder to 100% by weight of at least one solvent.

3. Use of at least one formulation according to either one of Claims 1 and 2 as corrosion inhibitor in any type of industry for the drilling of ores or fossil compounds, in particular in the oil and gas industries, and more generally as inhibitor of the corrosion of pipes in which crude oil or gas is transported.

4. Process for preventing or limiting the carbon dioxide corrosion and/or the hydrogen sulphide corrosion of metal parts, in particular of parts made of steel, capable of being damaged by carbon dioxide corrosion and/or by hydrogen sulphide corrosion, the said process comprising bringing the said metal parts into contact with at least one formulation according to either one of Claims 1 and 2.

5. Process according to Claim 4, in which the formulation comprising the carboxylate is injected continuously, batchwise or by squeeze injection into the fluids transported in the various pipes, valves, pumps, or the like, of a drilling installation, in particular for the drilling of oil or gas.

6. Process according to Claim 4 or Claim 5, in which the amount of imidazoline carboxylate is advantageously between 1 ppm and 10% (weight/volume), with respect to the fluid transported, preferably between 2 ppm and 50 ppm (weight/volume) for the continuous injection, between 100 ppm and 1% (weight/volume) for the batchwise treatment and from 1% to 10% (weight/volume) for the squeeze treatment.
